# EUROPÄISCHE PATENTANMELDUNG

(11) **EP 0 916 402 A1**
(43) Veröffentlichungstag der Anmeldung: **19.05.1999**
(21) Anmeldenummer: 98118418.7
(22) Anmeldetag: 29.09.1998
(51) Int. Cl.: B01J 21/08, B01J 35/10, B01J 37/00, C04B 35/14, C09C 1/30

(54) **Presslinge auf Basis von pyrogen hergestelltem Siliciumdioxid**

(30) Priorität: 13.11.1997 DE 19750238
(71) Anmelder: DEGUSSA AG, 60311 Frankfurt (DE)
(72) Erfinder: Krause, Helmfried, 63517 Rodenbach (DE); Rotgerink, Hermanus Lansink, Dr., 63864 Glattbach (DE); Feuer, Oliver, 61130 Nidderau (DE); Tacke, Thomas, Dr., 42001 Paducah, KY (US); Panster, Peter, Dr., 63517 Rodenbach (DE)

(57) **Zusammenfassung**

Preßlinge auf Basis von pyrogen hergestelltem Siliciumdioxid mit einem Porenvolumen von 0,5 bis 1,8 ml/g werden hergestellt, indem man pyrogen hergestelltes Siliciumdioxid mit Methylhydroxyethylcellulose, Wachs und Polyethylenglycol unter Zusatz von Wasser homogenisiert einem Knet- und Verformungsverfahren unterzieht, extrudiert, die Extrudate gegebenenfalls mittels einer Schneidvorrichtung auf die gewünschte Länge schneidet, bei einer Temperatur von 70 bis 150°C trocknet und während eines Zeitraums von 30 Minuten bis zu 10 Stunden bei einer Temperatur von 400 bis 1200°C tempert.

Diese Preßlinge können als Katalysator oder Katalysatorträger bei der Vinylacetatmonomerherstellung der Ethylenhydratisierung und Propylenhydratisierung eingesetzt werden.

## Beschreibung

Die Erfindung betrifft Preßlinge auf Basis von pyrogen hergestelltem Siliciumdioxid, ein Verfahren zu ihrer Herstellung und ihre Verwendung als Katalysatorträger oder Katalysator.

Pyrogen hergestellte Siliciumdioxide zeichnen sich durch extreme Feinteiligkeit und entsprechend hohe spezifische Oberfläche, sehr hohe Reinheit, sphärische Teilchenform und das Fehlen von Poren aus. Aufgrund dieser Eigenschaften finden die pyrogen hergestellten Oxide zunehmend Interesse als Träger für Katalysatoren (D. Koth, H. Ferch, Chem. Ing. Techn. 52, 628 (1980)).

Da pyrogen hergestellte Oxide besonders feinteilig sind, bereitet die Verformung zu Katalysatorträgern bzw. Katalysatoren einige Schwierigkeiten.

Aus der DE-A 31 32 674 ist ein Verfahren zur Herstellung von Preßlingen aus pyrogen hergestellten Oxiden bekannt, bei welchem Kieselsol als Bindemittel verwendet wird.

Aus der DE-A 34 06 185 ist ein Verfahren zur Herstellung von Preßlingen bekannt, bei welchem man Glasurfrittenpulver als Bindemittel und Glycerin als Gleitmittel verwendet.

Aus der DE-B 21 00 778 ist bekannt, Granulate auf Basis pyrogen hergestellter Siliciumdioxide bei der Herstellung von Vinylacetatmonomer als Katalysatorträger einzusetzen.

Aus der DE-A 39 12 504 ist ein Verfahren zur Herstellung von Preßlingen bekannt, bei dem man Aluminiumstearat, Magnesiumstearat und/oder Graphit als Gleitmittel und Harnstoff sowie Methylcellulose als Porenbildner verwendet.

Die bekannten mit Magnesiumstearat hergestellten Preßlinge werden als Aerosil-Tabletten Nr. 350, Firma Degussa, in den Handel gebracht. Sie weisen ca. 0,4 Gew.-% Mg auf.

Aus der EP-B 0 519 435 ist es bekannt, SiO₂ mittels Bindemittel zu Träger zu verpressen, die erhaltenen Träger zu glühen und die geglühten Trägerteilchen mittels Säure zu waschen, bis keine weiteren Kationen des Bindemittels abgegeben werden.

Die bekannten Verfahren haben den Nachteil, daß die erhaltenen Preßlinge für bestimmte katalytische Reaktionen, wie zum Beispiel die Vinylacetatherstellung aus Ethylen, Essigsäure und Sauerstoff, die Hydratisierung von Ethylen zu Ethanol oder die Hydratisierung von Propylen zu Isopropanol, nicht die gewünschten optimalen Eigenschaften, wie zum Beispiel eine hohe Reinheit, hohe Aktivität, hohe Selektivität, hohe Produktausbeute und hohe Stabilität aufweisen.

Die ältere aber nicht vorveröffentlichte Patentanmeldung DE 196 19 961.1-41 beschreibt Preßlinge auf Basis von pyrogen hergestelltem Siliciumdioxid mit den folgenden physikalisch-chemischen Kenndaten:

| | |
|---|---|
| Außendurchmesser | 0,8 - 20 mm |
| BET-Oberfläche | 30 - 400 m²/g |
| Porenvolumen | 0,5 - 1,3 ml/g |
| Bruchfestigkeit | 10 bis 250 N |
| Zusammensetzung | > 99,8 Gew.-% SiO₂ |
| Sonstige Bestandteile | < 0,2 Gew.-% |
| Abrieb | < 5 Gew.-% |
| Schüttgewicht | 350 - 750 g/l |

Gegenstand der Erfindung sind Preßlinge auf Basis von pyrogen hergestelltem Siliciumdioxid, welche dadurch gekennzeichnet sind, daß ein Porenvolumen von 0,5 bis 1,8 ml/g, vorzugsweise 1,31 bis 1,8 ml/g, aufweisen.

Die erfindungsgemäßen Preßlinge können einen Aussendurchmesser von 0,8 bis 20 mm, eine BET-Oberfläche von 30 bis 400 m²/g sowie eine Bruchfestigkeit von 7 bis 250 N aufweisen.

Vorzugsweise kann der SiO₂-Anteil der erfindungsgemäßen Preßlinge mehr als 99,0 Gew.-% betragen. Der Anteil an sonstigen Bestandteilen kann weniger als 0,2 Gew.-% betragen. Die erfindungsgemäßen Preßlinge können daher als frei von Bindemittel bezeichnet werden. Der Abrieb kann weniger als 5 Gew.-% betragen. Die Stampfdichte kann 300 bis 750 g/l betragen.

Ein weiterer Gegenstand der Erfindung ist ein Verfahren zur Herstellung von Preßlingen auf Basis pyrogen hergestelltem Siliciumdioxid mit einem Porenvolumen von 0,5 bis 1,8 ml/g, welches dadurch gekennzeichnet ist, daß man pyrogen hergestelltes Siliciumdioxid mit Methylhydroxyethylcellulose, Wachs und Polyethylenglycol unter Zusatz von Wasser homogenisiert, einem Knet- und Verformungsverfahren unterzieht, extrudiert, die Extrudate gegebenenfalls mittels einer Schneidvorrichtung auf die gewünschte Länge schneidet, bei einer Temperatur von 70 bis 150°C trocknet und während eines Zeitraumes von 30 Minuten bis zu 10 Stunden bei einer Temperatur von 400 bis 1200°C tempert.

Zur Durchführung des erfindungsgemäßen Verfahrens sind alle Kneter, Mischer oder Mühlen, die eine gute Homogenisierung und Verdichtung des Mischgutes ermöglichen, wie zum Beispiel Schaufel-, Wirbelschicht-, Kreisel- oder Luftstrommischer, geeignet. Besonders geeignet sind Mischer, mit denen eine zusätzliche Verdichtung des Mischgutes möglich ist, wie zum Beispiel Pflugmischer, Kollergänge oder Kugelmühlen. Die Mischung und Knetung kann aber auch direkt im Extruder erfolgen. Die Herstellung der Extrudate kann auf Ein- oder Zweischneckenextrudern, Strangpressen als auch auf Kompaktoren erfolgen. Vorzugsweise können die erfindungsgemäßen Preßlinge mittels Extrudern hergestellt werden.

Vor dem Verpressen kann in einer besonderen Ausführungsform der Erfindung die Mischung die folgende Zusammensetzung aufweisen:
- 50-90 Gew.-%: Siliciumdioxid, vorzugsweise 65-85 Gew.-%;
- 0,1-20 Gew.-%: Methylhydroxyethylcellulose, vorzugsweise 5-15 Gew.-%;
- 0,1-15 %: Wachs, vorzugsweise 5-12 Gew.-%;
- 0,1-15 %: Polyethylenglykol, vorzugsweise 5-10 Gew.-%

Die Preßlinge können verschiedene, zum Beispiel zylinderische, kugelförmige oder ringförmige Formen mit einem Außendurchmesser von 0,8 bis 20 mm aufweisen. Die Preßlinge werden bei 400 - 1200 °C 30 Minuten bis 10 Stunden getempert. Durch Variation der Einsatzstoffmengen und des Preßdruckes können die Bruchfestigkeit, die spezifische Gesamtoberfläche und das Porenvolumen in einem gewissen Rahmen eingestellt werden.

Die erfindungsgemäßen Preßlinge können entweder direkt als Katalysator oder als Katalysatorträger eingesetzt werden. In dem letzten Fall können die Preßlinge nach ihrer Herstellung mit einer katalytisch wirksamen Substanz in Kontakt gebracht und gegebenenfalls durch eine geeignete Nachbehandlung aktiviert werden.

Insbesondere lassen sich die Preßlinge aus pyrogen hergestelltem Siliciumdioxid als Träger für den Katalysator bei der Herstellung von Vinylacetatmonomer aus Ethylen, Essigsäure und Sauerstoff sowie als Katalysatorträger im Olefinhydratisierungsverfahren, zum Beispiel bei der Herstellung von Ethanol und Isopropanol, verwenden.

Die erfindungsgemäßen Preßlinge weisen die folgenden Vorteile auf:

Gegenüber den Preßlingen gemäß dem Dokument DE-A 39 12 504 weisen die erfindungsgemäßen Preßlinge außer Siliciumdioxid keine anderen anorganischen Bestandteile auf. Die bekannten Preßlinge haben den Nachteil, daß sie ca. 0,4 Gew.-% Mg enthalten, welches während des Verfahrens zur Hydratisierung von Olefinen ausgelaugt wird.

Die erfindungsgemäßen Preßlinge weisen dagegen eine verbesserte hydrothermale Stabilität bei derartigen Hydratisierungsverfahren auf. Zusätzlich weisen sie eine hohe Reinheit und ein hohes Porenvolumen auf.

Ein weiterer Vorteil ergibt sich daraus, daß eine größere Raum-Zeit-Ausbeute bei der Hydratisierung erzielt wird. Bei der Hydratisierung von Olefinen spielt das Porenvolumen des Katalysatorträgers eine sehr wichtige Rolle. Das größere Porenvolumen ermöglicht überraschenderweise die Aufnahme von mehr Aktivphase. Dies hat zur Folge, daß die Raum-Zeit-Ausbeute gesteigert wird.

Ein weiterer Gegenstand der Erfindung ist ein Trägerkatalysator für die Produktion von Vinylacetatmonomer (VAM), welcher auf einem Träger (Preßling) als katalytisch aktive Komponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, welches dadurch gekennzeichnet ist, daß der Träger ein erfindungsgemäßer Preßling ist. Als Alkaliverbindungen sind Kaliumverbindungen, wie z. B. Kaliumacetat, bevorzugt.

Die katalytisch aktiven Komponenten können in folgenden Systemen vorhanden sein:
Pd/Au/Alkali-Verbindungen
Pd/Cd/Alkali-Verbindungen
Pd/Ba/Alkali-Verbindungen

Die erfindungsgemäßen Trägerkatalysatoren werden für die Produktion von Vinylacetatmonomer verwendet. Dazu werden Ethen, Essigsäure und molekularer Sauerstoff bzw. Luft in der Gasphase, gegebenenfalls unter Zusatz von Inertgasen, bei Temperaturen zwischen 100 und 250 °C und gewöhnlichem oder erhöhtem Druck in Gegenwart des erfindungsgemäßen Trägerkatalysators zur Reaktion gebracht.

Ein derartiges Produktionsverfahren ist aus den Dokumenten DE 16 68 088, US 4,048,096, EP-A 0 519 435, EP-A 0 634 208, EP-A 0 723 810, EP-A 0 634 209, EP-A 0 632 214 und EP-A 0 654 301 bekannt. Diese Patentschriften offenbaren auch ein Verfahren zur Herstellung von Trägerkatalysatoren. Je nach Ausführungsform werden Trägerkatalysatoren mit homogener Edelmetallverteilung über den Trägerquerschnitt und mit mehr oder weniger ausgeprägtem Schalenprofil erhalten.

Ein weiterer Gegenstand der Erfindung ist ein Verfahren zur Herstellung des Trägerkatalysators für die Produktion von Vinylacetatmonomer durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, welches dadurch gekennzeichnet ist, daß der Träger ein Preßling gemäß Erfindung ist.

Ein weiterer Gegenstand der Erfindung ist ein Verfahren zur Herstellung des Trägerkatalysators für die Produktion von Vinylacetatmonomer durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, welches dadurch gekennzeichnet ist, daß der Träger ein Preßling gemäß Erfindung ist.

Die erfindungsgemäßen Trägerkatalysatoren können zur Herstellung ungesättigter Ester aus Olefinen, Säuren und Sauerstoff in der Gasphase eingesetzt werden.

Die erfindungsgemäßen Katalysatoren des Katalysatorsystems Pd/Alkali/Au werden gewöhnlich durch Imprägnieren der Träger mit einer basischen Lösung und einer Gold- und Palladiumsalze enthaltenden Lösung erhalten, wobei die Imprägnierschritte gleichzeitig oder nacheinander, mit oder ohne Zwischentrocknung erfolgen. Anschließend wird der Träger zur Entfernung gegebenenfalls vorhandener Chloridanteile gewaschen. Vor oder nach dem Waschen werden die auf dem Träger ausgefällten unlöslichen Edelmetallverbindungen reduziert. Die so erhaltene Katalysatorvorstufe wird getrocknet und zur Aktivierung des Katalysators mit Alkaliacetaten oder Alkaliverbindungen, die sich unter den Reaktionsbedingungen bei der Produktion von Vinylacetatmonomer ganz oder teilweise in Alkaliacetate umwandeln, imprägniert. Im allgemeinen liegen die Edelmetalle bei Pd/Au-Katalysatoren in Form einer Schale auf dem Träger vor.

Bei Pd/Alkali/Ba-Katalysatoren werden die Metallsalze durch Tränken, Aufsprühen, Aufdampfen, Tauchen oder Ausfällen aufgebracht (EP 0 519 436). Dieselben Methoden sind bei Pd/Alkali/Cd-Katalysatoren bekannt (US-PS 4,902,823, US-PS 3,393,199, US-PS 4,668,819).

Je nach Katalysatorsystem kann eine Reduktion des Trägerkatalysators vorgenommen werden.

Die Reduktion des Katalysators kann in der wäßrigen Phase oder in der Gasphase vorgenommen werden. Zur Reduktion in der wäßrigen Phase eignen sich zum Beispiel Formaldehyd oder Hydrazin. Die Reduktion in der Gasphase kann mit Wasserstoff beziehungsweise Formiergas (95 Vol.-% N₂ + 5 Vol.-% H₂) Ethen oder stickstoffverdünntem Ethen vorgenommen werden. Gemäß der EP 0 634 209 erfolgt die Reduktion mit Wasserstoff bei Temperaturen zwischen 40 und 260 °C, bevorzugt zwischen 70 und 200 °C. Gemäß der EP-A 0 723 810 erfolgt die Reduktion mit Formiergas (95 Vol.-% N₂ und 5 Vol.-% H₂) bei Temperaturen zwischen 300 und 550 °C, bevorzugt zwischen 350 und 500 °C. Häufig wird der Katalysator jedoch erst nach der Aktivierung mit Alkaliacetat direkt im Produktionsreaktor mit Ethen reduziert.

Im Produktionsprozeß wird der Katalysator erst langsam mit den Reaktanden belastet. Während dieser Anfahrphase erhöht sich die Aktivität des Katalysators und erreicht gewöhnlich erst nach Tagen oder Wochen ihr endgültiges Niveau.

Aufgabe der vorliegenden Erfindung ist es, einen Trägerkatalysator für die Produktion von Vinylacetatmonomer anzugeben, welcher bei gleicher beziehungsweise verbesserter Selektivität eine höhere Aktivität als bekannte Katalysatoren aufweist.

Gegenstand der Erfindung ist ein Trägerkatalysator, der auf dem erfindungsgemäßen Träger aus Siliziumdioxid als katalytisch aktive Komponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, sowie ein Verfahren zu seiner Herstellung.

Als Trägermaterial für den Katalysator eignet sich der erfindungsgemäße Preßling auf Basis von pyrogen hergestelltem Siliciumdioxid. Wichtig ist, daß die Katalysatorträger unter den Reaktionsbedingungen des katalytischen Prozesses, insbesondere unter dem Einfluß von Essigsäure, ihre mechanische Festigkeit behalten.

Die erfindungsgemäßen Preßlinge können als Strangpreßlinge, Tabletten, Ringe oder in anderen für Festbettkatalysatoren üblichen Formen vorliegen.

Im folgenden wird die Herstellung von Trägerkatalysatoren des Systems Pd/Alkali/Au auf dem erfindungsgemäßen Preßling näher beschrieben.

Die erfindungsgemäßen Preßlinge werden mit einer Palladium und Gold enthaltenden Lösung imprägniert. Gleichzeitig mit der edelmetallhaltigen Lösung oder in beliebiger Reihenfolge nacheinander werden die erfindungsgemäßen Preßlinge mit einer basischen Lösung imprägniert, welche ein oder mehrere basische Verbindungen enthalten kann. Die basische Verbindung oder Verbindungen dienen zur Überführung des Palladiums und Golds in ihre Hydroxide.

Die Verbindungen in der basischen Lösung können aus Alkalihydroxiden, Alkalibicarbonaten, Alkalicarbonaten, Alkalisilikaten oder Mischungen dieser Stoffe bestehen. Bevorzugt werden Kaliumhydroxid und/oder Natriumhydroxid verwendet.

Zur Herstellung der edelmetallhaltigen Lösung können als Palladiumsalze beispielsweise Palladiumchlorid, Natrium- bzw. Kaliumpalladiumchlorid oder Palladiumnitrat verwendet werden. Als Goldsalze eignen sich Gold(III)-chlorid und Tetrachlorogold(III)-säure. Vorzugsweise können Kaliumpalladiumchlorid, Natriumpalladiumchlorid und/oder Tetrachlorogoldsäure eingesetzt werden.

Das Imprägnieren der erfindungsgemäßen Preßlinge mit der basischen Lösung beeinflußt die Abscheidung der Edelmetalle in dem Preßling. Die basische Lösung kann entweder gleichzeitig mit der Edelmetallösung oder in beliebiger Reihenfolge mit dieser Lösung mit dem erfindungsgemäßen Preßling in Kontakt gebracht werden. Bei aufeinanderfolgender Imprägnierung des erfindungsgemäßen Preßlings mit den beiden Lösungen kann nach dem ersten Imprägnierschritt eine Zwischentrocknung vorgenommen werden.

Bevorzugt wird der erfindungsgemäße Preßling zuerst mit der basischen Verbindung imprägniert. Die anschließende Imprägnierung mit der Palladium und Gold enthaltenden Lösung führt zur Ausfällung von Palladium und Gold in einer oberflächlichen Schale auf dem erfindungsgemäßen Preßling. Die umgekehrte Reihenfolge der Imprägnierungen führt im allgemeinen zu einer mehr oder weniger homogenen Verteilung der Edelmetalle über den Querschnitt des erfindungsgemäßen Preßlings. Bei geeigneter Verfahrensführung können jedoch auch bei umgekehrter Imprägnier-Reihenfolge Katalysatoren mit definierter Schale erhalten werden (siehe z. B. US 4,048,096). Katalysatoren mit homogener oder nahezu homogener Edelmetall-Verteilung weisen im allgemeinen eine geringere Aktivität und Selektivität auf.

Katalysatoren mit Schalendicken von unter 1 mm, bevorzugt von etwa unter 0,5 mm, sind besonders geeignet. Die Schalendicke wird durch die Menge der auf das Trägermaterial aufgebrachten basischen Verbindung relativ zur gewünschten Menge der Edelmetalle beeinflußt. Je höher dieses Verhältnis ist, desto geringer wird die Dicke der sich ausbildenden Schale. Das für eine gewünschte Schalendicke erforderliche Mengenverhältnis von basischer Verbindung zu den Edelmetallverbindungen hängt von der Beschaffenheit des Trägermaterials sowie von der gewählten basischen Verbindung und den Edelmetallverbindungen ab. Das erforderliche Mengenverhältnis wird zweckmäßigerweise durch wenige Vorversuche ermittelt. Die sich ergebende Schalendicke kann dabei in einfacher Weise durch aufschneiden der Katalysatorteilchen ermittelt werden.

Die minimal notwendige Menge der basischen Verbindung ergibt sich aus der stöchiometrisch berechneten Menge an Hydroxidionen, die zur Überführung des Palladiums und Golds in die Hydroxide benötigt werden. Als Richtwert gilt, daß die basische Verbindung für eine Schalendicke von 0,5 mm in einem 1 bis 10-fachen stöchiometrischen Überschuß angewendet werden sollte.

Die erfindungsgemäßen Preßlinge werden nach dem Verfahren der Porenvolumenimprägnierung mit den basischen Verbindungen und den Edelmetallsalzen belegt. Wird mit Zwischentrocknung gearbeitet, wählt man die Volumina der beiden Lösungen so, daß sie jeweils etwa 90 bis 100 % der Aufnahmekapazität der erfindungsgemäßen Preßlinge entsprechen. Wird auf die Zwischentrocknung verzichtet, so muß die Summe der Einzelvolumina der beiden Imprägnierlösungen der obigen Bedingung entsprechen, wobei die Einzelvolumina im Verhältnis von 1 : 9 bis 9 : 1 zueinander stehen können. Bevorzugt wird ein Volumenverhältnis von 3 : 7 bis 7 : 3, insbesondere von 1 : 1, angewendet. Als Lösungsmittel kann in beiden Fällen bevorzugt Wasser verwendet werden. Es können aber auch geeignete organische oder wäßrig-organische Lösungsmittel eingesetzt werden.

Die Umsetzung der Edelmetallsalzlösung mit der basischen Lösung zu unlöslichen Edelmetallverbindungen erfolgt langsam und ist je nach Präparationsmethode im allgemeinen erst nach 1 bis 24 Stunden abgeschlossen. Danach werden die wasserunlöslichen Edelmetallverbindungen mit Reduktionsmitteln behandelt. Es kann eine Naßreduktion zum Beispiel mit wäßrigem Hydrazinhydrat oder eine Gasphasenreduktion mit Wasserstoff, Ethen, Formiergas oder auch Methanoldämpfen vorgenommen werden. Die Reduktion kann bei Normaltemperatur oder erhöhter Temperatur und bei Normaldruck oder erhöhtem Druck erfolgen; gegebenenfalls auch unter Zugabe von Inertgasen.

Vor und/oder nach der Reduktion der Edelmetallverbindungen wird das auf dem Träger gegebenenfalls vorhandene Chlorid durch eine gründiche Waschung entfernt. Nach der Waschung sollte der Katalysator weniger als 500, besser weniger als 200 ppm, Chlorid enthalten.

Die nach der Reduktion erhaltene Katalysatorvorstufe wird getrocknet und abschließend mit Alkaliacetaten oder Alkaliverbindungen, die sich unter den Reaktionsbedingungen bei der Produktion von Vinylacetatmonomer ganz oder teilweise in Alkaliacetate umwandeln, imprägniert. Vorzugsweise wird mit Kaliumacetat imprägniert. Hierbei wird wieder bevorzugt die Porenvolumenimprägnierung angewendet. Das heißt: Die benötigte Menge an Kaliumacetat wird in einem Lösungsmittel, vorzugsweise Wasser, dessen Volumen etwa der Aufnahmekapazität des vorgelegten Trägermaterials für das gewählte Lösungsmittel entspricht, gelöst. Dieses Volumen ist etwa gleich dem Gesamtporenvolumen des Trägermaterials.

Der fertige Katalysator wird anschießend bis auf eine Restfeuchte von weniger als 2 % getrocknet. Die Trocknung kann an Luft, gegebenenfalls auch unter Stickstoff als Inertgas, erfolgen.

Die Herstellung von Trägerkatalysatoren der Systeme Pd/Alkali/Cd bzw. Pd/Alkali/Ba auf den erfindungsgemäßen Preßlingen erfolgt nach den oben zitierten Patentschriften.

Für die Synthese von Vinylacetatmonomer ist es zweckmäßig, den Katalysator mit 0,2 bis 4, vorzugsweise 0,3 bis 3 Gew.-% Palladium, 0,1 bis 2, vorzugsweise 0,15 bis 1,5 Gew.-% Gold und 1 bis 10, vorzugsweise 1,5 bis 9 Gew.-% Kaliumacetat, jeweils bezogen auf das Gewicht des eingesetzten Trägers, zu belegen. Diese Angaben gelten für das System Pd/Alkali/Au. Im Fall von Katalysatorträgern mit einer Schüttdichte von 500 g/l entsprechen diese Konzentrationsangaben volumenbezogenen Konzentrationen von 1,0 bis 20 g/l Palladium, 0,5 bis 10 g/l Gold und 5 bis 50 g/l Kaliumacetat. Zur Anfertigung der Imprägnierlösungen werden die entsprechenden Mengen der Palladium- und Goldverbindungen in einem Volumen Wasser, welches etwa 90 bis 100 % der Wasseraufnahmekapazität des vorgelegten Trägermaterials entspricht, gelöst. Ebenso wird bei der Anfertigung der basischen Lösung verfahren.

Der Cadmium-Gehalt der Pd/Alkali/Cd-Katalysatoren beträgt im allgemeinen 0,1 bis 2,5 Gew.-%, vorzugsweise 0,4 bis 2,0 Gew.-%.

Der Barium-Gehalt der Pd/Alkali/Ba-Katalysatoren beträgt im allgemeinen 0,1 bis 2,0 Gew.-%, vorzugsweise 0,2 bis 1,8 Gew.-%.

Der Palladium-Gehalt der Pd/Alkali/Cd beziehungsweise Pd/Alkali/Ba-Katalysatoren kann 0,2 bis 4 Gew.-%, bevorzugt 0,3 bis 3 Gew.-% Palladium betragen.

Der Kalium-Gehalt der Pd/Alkali/Cd- bzw. Pd/Alkali/Ba-Katalysatoren beträgt im allgemeinen 1 bis 10 Gew.-%, vorzugsweise 1,5 bis 9 Gew.-%.

Die Erfindung betrifft weiterhin katalytische Umsetzungen unter hydrothermalen Bedingungen, wie z. B. die Hydratisierung von Olefinen zu den entsprechenden Alkoholen in Gegenwart von Phosphorsäure oder einer anderen Aktivkomponente, zum Beispiel eine Heteropolysäure, wobei der erfindungsgemäße Preßling als Katalysatorträger dient.

Ein solches Verfahren wird zum Beispiel in der EP 0 578 441 A2 beschrieben. Nach diesem Verfahren wird Wasser und Ethylen bei Temperaturen zwischen 225 und 280°C und Drucken zwischen 20 und 240 bar zu Ethanol umgesetzt. Dabei wird ein Wasser/Ethylen-Molverhältnis im Bereich von 0,15 bis 0,5 angewendet. Die Katalysatorbelastung, gemessen in Gramm Wasser/Ethylen-Gemisch pro Minute und Milliliter Katalysator, kann im Bereich von 0,01 bis 0,1 g/(min × ml) gewählt werden. Als Nebenprodukt wird bei dieser Reaktion Diethylether gebildet.

Die Verfahrensparameter zur Herstellung von Ethanol können auch außerhalb der obenstehenden Bereiche liegen. Ein Beispiel hiervon findet man in DE-OS 20 15 536 (Beispiel 1): Die Temperatur betrug in diesem Fall etwa 300 °C, und das Wasser zu Ethylen-Verhältnis war 0,74 Mol/Mol, bei einem Gesamtdruck von 70 bar. Das wesentliche ist hier, daß Temperatur und Wasserdampfdruck aufeinander abgestimmt sein müssen.

Die Herstellung von Isopropanol durch Hydratisierung von Propylen erfolgt unter ähnlichen Bedingungen. Dabei liegt die Temperatur jedoch im Bereich zwischen 180 und 225°C. Als Nebenprodukt entsteht bei dieser Reaktion n-Propanol.

Als Katalysatorträger für die Aktivkomponente Phosphorsäure können gemäß EP 0 578 441 A2 Pellets aus synthetischem Siliziumdioxid mit hoher Bruchfestigkeit, hoher Porosität und geringen metallischen Verunreinigungen eingesetzt werden. Die Poren des Trägers dienen zur Aufnahme der Aktivkomponente. Der mittlere Porenradius vor dem Einsatz im Hydratisierungsprozeß liegt im Bereich zwischen 1 und 50 nm.

Im Falle der Hydratisierung von Olefinen wird Phosphorsäure als Aktivkomponente in den Katalysatorträger eingebracht. Hierzu wird der Träger in eine wäßrige Lösung von Phosphorsäure getaucht und mit dieser getränkt. Zur Anwendung kommen hierbei Phosphorsäurelösungen mit 15 bis 85 Gew.-% Phosphorsäure bezogen auf das Gesamtgewicht der Lösung. Auch die Belegung mit einer Heteropolysäure als Aktivkomponente ist möglich.

Ein Hauptanwendungsgebiet der Hydratisierung von Olefinen ist die Hydratisierung von Ethylen zur Herstellung von Ethanol und Diethylether sowie die Hydratisierung von Propylen zur Herstellung von Isopropanol. Es werden dabei die aus dem Stand der Technik bekannten Reaktionsbedingungen angewendet.

Als pyrogen hergestelltes Siliciumdioxid können Siliciumdioxide mit den folgenden physikalisch-chemischen Kenndaten eingesetzt werden:

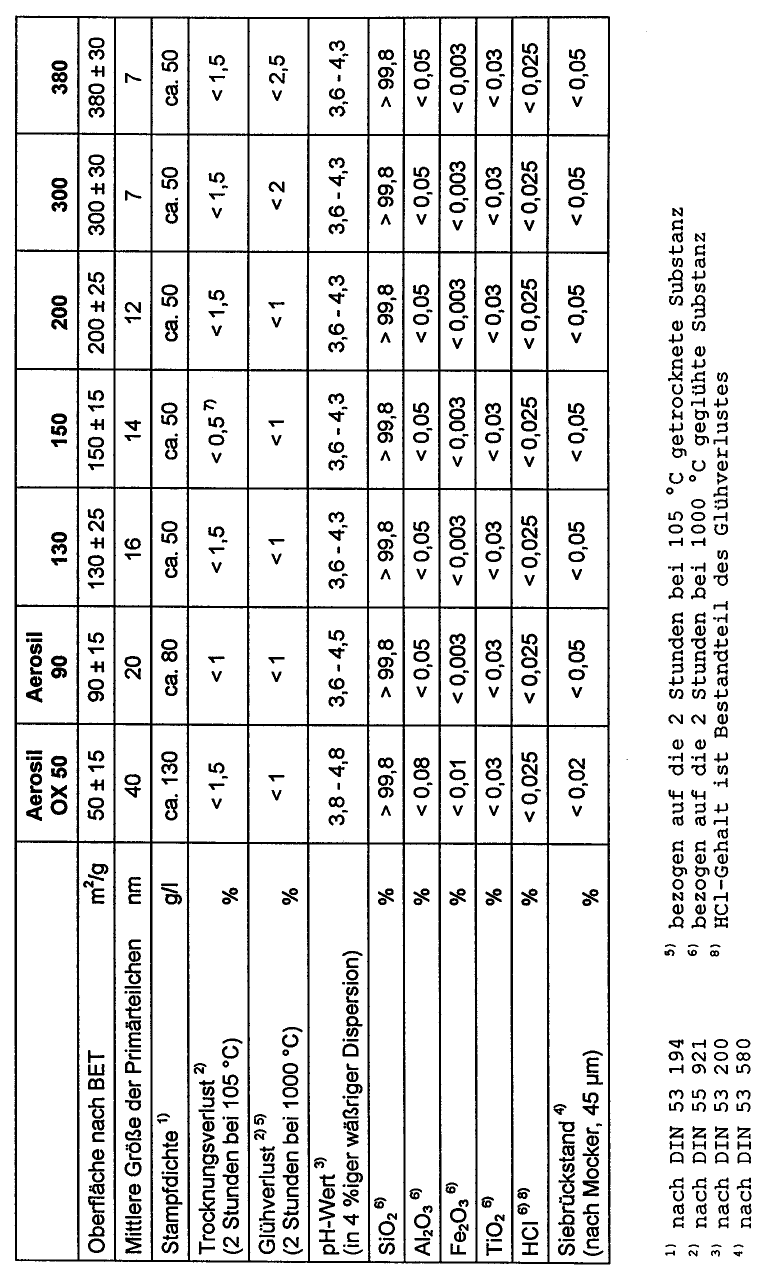

Zur Herstellung von AEROSIL wird in eine Knallgasflamme aus Wasserstoff und Luft eine flüchtige Siliciumverbindung eingedüst. In den meisten Fällen verwendet man Siliciumtetrachlorid. Diese Substanz hydrolysiert unter dem Einfluß des bei der Knallgasreaktion entstehenden Wassers zu Siliciumdioxid und Salzsäure. Das Siliciumdioxid tritt nach dem Verlassen der Flamme in eine sogenannte Koagulationszone ein, in der die AEROSIL-Primärteilchen und -Primäraggregate agglomerieren. Das in diesem Stadium als eine Art Aerosol vorliegende Produkt wird in Zyklonen von den gasförmigen Begleitsubstanzen getrennt und anschließend mit feuchter Heißluft nachbehandelt. Durch dieses Verfahren laßt sich der Rest-Salzsäuregehalt unter 0,025 % senken. Da das AEROSIL am Ende dieses Prozesses mit einer Schüttdichte von nur ca. 15 g/l anfällt, wird eine Vakuumverdichtung, mit der sich Stampfdichten von ca. 50 g/l und mehr einstellen lassen, angeschlossen.

Die Teilchengrößen der auf diese Weise gewonnenen Produkte können mit Hilfe der Reaktionsbedingungen, wie zum Beispiel die Flammentemperatur, der Wasserstoff- oder Sauerstoffanteil, die Siliciumtetrachloridmenge, die Verweilzeit in der Flamme oder die Länge der Koagulationsstrecke, variiert werden.

Die BET-Oberfläche wird gemäß DIN 66 131 mit Stickstoff bestimmt. Das Porenvolumen wird rechnerisch aus der Summe von Mikro-, Meso- und Makroporenvolumen bestimmt. Die Bruchfestigkeit wird mittels des Bruchfestigkeitstesters der Firma Erweka, Typ TBH 28, bestimmt.

Die Bestimmung der Mikro- und Mesoporen erfolgt durch Aufnahme einer N₂-Isotherme und deren Auswertung nach BET, de Boer und Barret, Joyner, Halenda.

Die Bestimmung der Makroporen erfolgt durch das Hg-Einpreßverfahren.

Der Abrieb wird mittels des Abrieb- und Friabilitätstesters der Firma Erweka, Typ TAR, bestimmt.

### Beispiele

### Beispiel 1

- 69 Gew.-%: Aerosil 200
- 12,5 Gew.-%: Methylhydroxyethylcellulose
- 10,2 Gew.-%: Wachs
- 8,3 Gew.-%: Polyethylenglycol
werden unter Zusatz von Wasser in einem Kneter kompaktiert. Die Knetmasse wird in einem Einschneckenextruder zu Strangpreßlingen geformt und mit einer Schneidvorrichtung auf die gewünschte Länge von 3 bis 5 mm geschnitten. Die Preßlinge werden in einem Bandtrockner bei 90°C getrocknet. Die Rohpreßlinge werden 6 Stunden bei 750°C calcinert.

Die erhaltenen Preßlinge weisen folgende physikalischchemischen Kenndaten auf:

| Preßlingsabmessungen: | |
|---|---|
| Durchmesser (mm) | 3,75 |
| Länge (mm) | 4 ± 1 |
| BET-Oberfläche (m²/g) | 160 |
| Porenvolumen (ml/g) | 1,41 |
| Bruchfestigkeit (N) | 25 |
| Abrieb (Gew.-%) | 2,1 |
| Stampfdichte (g/l) | 348 |
| SiO₂-Gehalt (Gew.-%) | 99,85 |

### Beispiel 2 und 3

- 71,4 Gew.-%: Aerosil 200
- 12,9 Gew.-%: Methylhydroxyethylcellulose
- 7,1 Gew.-%: Wachs
- 8,6 Gew.-%: Polyethylenglycol
werden unter Zusatz von Wasser in einem Zweischneckenextruder zu Strangpreßlingen geformt und mit einer Schneidvorrichtung auf die gewünschte Länge von 3 bis 5 bzw. 2 bis 4 mm geschnitten. Die Preßlinge werden bei 110°C in einem Trockenschrank getrocknet. Die Rohpreßlinge werden 6 Stunden bei 750°C calcinert.

Die erhaltenen Preßlinge weisen folgende physikalischchemischen Kenndaten auf:

| | Beispiel 2 | Beispiel 3 |
|---|---|---|
| Preßlingsabmessungen: | | |
| Durchmesser (mm) | 3,7 | 2,65 |
| Länge (mm) | 4 ± 1 | 3 ± 1 |
| BET-Oberfläche (m²/g) | 160 | 163 |
| Porenvolumen (ml/g) | 1,46 | 1,54 |
| Bruchfestigkeit (N) | 20 | 7 |
| Abrieb (Gew.-%) | 0,8 | 1,15 |
| Stampfdichte (g/l) | 327 | 310 |
| SiO₂-Gehalt (Gew.-%) | 99,9 | 99,9 |

### Beispiel 4

### Hydratisierung von Ethylen unter Einsatz eines bekannten Katalysatorträgers

Der dem Stand der Technik entsprechende Katalysatorträger 350 von der Firma Degussa AG hat ein Porenvolumen von 0,8 ml/g. Dieser Katalystorträger wird in eine 60 Gew.-% H₃PO₄-Lösung getränkt und anschließend getrocknet. 50 ml dieses Katalysators wird in einem Festbettreaktor eingebaut und unter einem N₂-Gasstrom auf 240 °C hochgeheizt.
Anschließend wird N₂ durch ein gasförmiges Gemisch aus Ethylen und Wasser ersetzt. Der Wasserdurchsatz beträgt 0,20 g/min, das Wasser/Ethylen-Verhältnis ist 0,27 Mol/Mol. Die Analyse der Abgase erfolgt mittels on-line-Gaschromatographie. Zur Beurteilung der katalytischen Aktivität wird das Flächenverhältnis Ethanol zu Ethylen ausgewertet. Bei einer gemittelten Katalysatortemperatur von 240 °C wird für den Katalysator auf Basis des bekannten Träger 350 ein Verhältnis von 0,078 erreicht.

### Beispiel 5

### Hydratisierung von Ethylen unter Einsatz eines erfindungsgemäßen Katalysatorträgers

Ein erfindungsgemäßer SiO₂-Katalysatorträger mit einem Porenvolumen von 1,36 ml/g wird wie der Katalysatorträger gemäß Beispiel 4 mit H₃PO₄-Lösung beladen und getestet. Der Wasserdurchsatz beträgt 0,22 g/min, das Wasser zu Ethylen-Verhältnis ist 0,30 Mol/Mol. Bei diesem Katalysator auf dem erfindungsgemäßen Katalysatorträger beträgt das Gaschromatographie-Flächenverhältnis 0,103, d. h., es ist 32 % höher als bei dem Katalysator auf dem bekannten Träger.

## Patentansprüche

1. Preßlinge auf Basis von pyrogen hergestelltem Siliciumdioxid, gekennzeichnet durch ein Porenvolumen von 0,5 bis 1,8 ml/g.

2. Verfahren zur Herstellung der Preßlinge auf Basis von pyrogen hergestelltem Siliciumdioxid mit einem Porenvolumen von 0,5 bis 1,8 ml/g gemäß Anspruch 1, dadurch gekennzeichnet, daß man pyrogen hergestelltes Siliciumdioxid mit Methylhydroxyethylcellulose, Wachs und Polyethylenglycol unter Zusatz von Wasser homogenisiert, einem Knet- und Verformungsverfahren unterzieht, extrudiert, die Extrudate gegebenenfalls mittels einer Schneidvorrichtung auf die gewünschte Länge schneidet, bei einer Temperatur von 70 bis 150°C trocknet und während eines Zeitraums von bis zu 10 Stunden bei einer Temperatur von 400 bis 1200°C tempert.

3. Verwendung der Preßlinge gemäß Anspruch 1 als Katalysator oder Katalysatorträger.

4. Trägerkatalysator für die Produktion von Vinylacetatmonomer (VAM), welcher auf einem Träger (Preßling) als katalytisch aktive Komponenten Palladium und/oder dessen Verbindungen und Alkaliverbindungen, sowie zusätzlich Gold und/oder dessen Verbindungen (System Pd/Alkali/Au) oder Cadmium und/oder dessen Verbindungen (System Pd/Alkali/Cd) oder Barium und/oder dessen Verbindungen (System Pd/Alkali/Ba) oder Palladium, Alkaliverbindungen und Mischungen aus Gold und/oder Cadmium und/oder Barium enthält, dadurch gekennzeichnet, daß der Träger ein Preßling gemäß Anspruch 1 ist.

5. Verfahren zur Herstellung des Trägerkatalysators nach Anspruch 4 für die Produktion von Vinylacetatmonomer durch Tränken, Aufsprühen, Aufdampfen, Tauchen oder Ausfällen der Pd-, Au, Cd-, Ba-Metallverbindungen, gegebenenfalls Reduzieren der auf dem Träger aufgebrachten reduzierbaren Metallverbindungen, Waschen zur Entfernung gegebenenfalls vorhandener Chloridanteile, Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, in geeigneter Reihenfolge, dadurch gekennzeichnet, daß der Träger ein Preßling gemäß Anspruch 1 ist.

6. Verfahren zur Herstellung des Trägerkatalysators nach Anspruch 4 für die Produktion von Vinylacetatmonomer durch Imprägnieren des Trägers mit einer basischen Lösung und einer Gold- und Palladiumsalze enthaltenden Lösung, wobei die Imprägnierung gleichzeitig oder nacheinander, mit oder ohne Zwischentrocknung erfolgt, Waschen des Trägers zur Entfernung gegebenenfalls vorhandener Chloridanteile und Reduzieren der auf dem Träger ausgefällten unlöslichen Verbindungen vor oder nach dem Waschen, Trocknen der so erhaltenen Katalysatorvorstufe, und Imprägnieren mit Alkaliacetaten oder Alkaliverbindungen, die sich unter den Reaktionsbedingungen bei der Produktion von Vinylacetatmonomer ganz oder teilweise in Alkaliacetate umwandeln, dadurch gekennzeichnet, daß der Träger ein Preßling gemäß Anspruch 1 ist.

7. Trägerkatalysator nach Anspruch 4 bis 6, dadurch gekennzeichnet, daß es sich bei dem Alkaliacetat bzw. der Alkaliverbindung um Kaliumacetat handelt.

8. Verwendung des Katalysators nach Anspruch 4 bis 6 zur Herstellung ungesättigter Ester aus Olefinen, organischen Säuren und Sauerstoff in der Gasphase.

9. Verwendung von Katalysatoren, welche eine Aktivkomponente enthalten, die auf einem Preßling gemäß Anspruch 1 aufgebracht ist, für katalytische Umsetzungen unter hydrothermalen Bedingungen.

10. Verwendung nach Anspruch 9 für die Hydratisierung von Olefinen.

11. Verwendung nach Anspruch 10, dadurch gekennzeichnet, daß als Aktivkomponente Phosphorsäure verwendet wird.

12. Verwendung nach Anspruch 10, dadurch gekennzeichnet, daß als Aktivkomponente eine Heteropolysäure aufgebracht ist.

13. Verwendung nach Anspruch 11 oder 12 für die Hydratisierung von Ethylen zur Herstellung von Ethanol und Diethylether.

14. Verwendung nach Anspruch 11 oder 12 für die Hydratisierung von Propylen zur Herstellung von Isopropanol.
